Europäisches Patentamt

(19) European Patent Office

Office européen des brevets

(11) **EP 1 270 655 B1**

(12) **EUROPÄISCHE PATENTSCHRIFT**

(45) Veröffentlichungstag und Bekanntmachung des Hinweises auf die Patenterteilung:
**11.08.2004 Patentblatt 2004/33**

(51) Int Cl.$^7$: **C08J 11/14**, C07C 209/62, C07C 209/84, C07C 209/86, C07C 29/09, C07C 29/80 // C08L75/04

(21) Anmeldenummer: **02010645.6**

(22) Anmeldetag: **11.05.2002**

(54) **Verfahren zur Spaltung von Polyurethanen**

Process for the cleavage of polyurethanes

Procédé de décomposition de polyuréthanes

(84) Benannte Vertragsstaaten:
**AT BE CH CY DE DK ES FI FR GB GR IE IT LI LU MC NL PT SE TR**

(30) Priorität: **26.06.2001 DE 10130820**

(43) Veröffentlichungstag der Anmeldung:
**02.01.2003 Patentblatt 2003/01**

(73) Patentinhaber: **Degussa AG**
**40474 Düsseldorf (DE)**

(72) Erfinder: **Pinske, Klaus, Dr.**
**45721 Haltern (DE)**

(56) Entgegenhaltungen:
**DE-C- 951 268 US-A- 3 441 616**
**US-A- 4 035 314 US-A- 5 208 379**

EP 1 270 655 B1

**Beschreibung**

Die Erfindung betrifft ein Verfahren zur Spaltung von Polyurethanen.

Mit dem Wachstum der Polyurethan-erzeugenden Industrie und dem vielfältigen Einsatz von Polyurethanen stellt sich auch das Problem der Entsorgung von Polyurethanabfällen. Aus ökologischen und ökonomischen aber auch politischen Gründen wird als vorrangiges Ziel eine stoffliche Wiederverwendung angestrebt.

Verfahren zur stofflichen Verwertung von Polyurethanen sind in der Literatur zahlreich beschrieben und werden sowohl in Versuchs- als auch produktionstechnischen Anlagen bereits durchgeführt. Es handelt sich dabei um Reaktionen, bei denen die Urethan-Bindungen gespalten werden.

Das am häufigsten angewendete Verfahren ist die Glykolyse, bei dem das Polyurethan mit einem Glykol oder Glykolgemisch, bevorzugt mit Ethylen-, Diethylen-, Propylenglykol, zu einem kürzerkettigen Urethan gespalten wird (EP 0 835 901 A2, EP 0 546 415 B1, EP 0 899 292 A1, EP 0 546 415 A1, DE 4 442 379 A1, EP 0 771 644 A2):

$$- [-R\text{-}NH\text{-}(C{=}O)\text{-}O\text{-}R^1\text{-}(OR^2)_m\text{-}O\text{-}]_n\text{-} + nHO\text{-}R^3\text{-}OH \rightarrow n\ R\text{-}NH\text{-}(C{=}O)\text{-}O\text{-}R^3\text{-}OH + n\ HO\text{-}R^1\text{-}(OR^2)_m\text{-}OH$$

$R$         (cyclo)aliphatischer, aromatischer Kohlenwasserstoff
$R^1, R^2, R^3$     Alkylen

Der Reaktionsansatz mit dem kürzerkettigen und aufgrund der freien Hydroxylgruppe reaktiven Urethan und der abgespaltenen Polyol-Komponente wird anschließend, in der Regel ohne weitere Aufarbeitung, mit einer Isocyanat-Komponente wieder polyurethanisiert. Es entstehen dabei Misch-Polyurethane, die für neue, in der Regel weniger leistungsfähige Anwendungen eingesetzt werden. Damit verändert sich mit jedem Recyclingdurchgang auch die Produktzusammensetzung, was sich erschwerend auf die Einsatzmöglichkeiten auswirkt.

Die glykolytische Spaltung wird eingesetzt, wenn die einzelnen Komponenten des zu spaltenden Polyurethans nicht zurückgewonnen werden sollen.

Ein weiteres Verfahren zur Spaltung von Polyurethanen, das diesen Gesichtspunkt berücksichtigt, ist die Hydrolyse. Als Spaltungsprodukte entstehen bei der Reaktion des Polyurethans mit Wasser neben Kohlendioxid Amin- und Alkohol-Komponenten:

$$-[-R\text{-}NH\text{-}(C{=}O)\text{-}O\text{-}R^1\text{-}(OR^2)_m\text{-}O\text{-})_n\text{-} + n\ H_2O \rightarrow n\ R\text{-}NH_2 + n\ CO_2 + n\ HO\text{-}R^1\text{-}(OR^2)_m\text{-}OH$$

$R$         (cyclo)aliphatischer, aromatischer Kohlenwasserstoff
$R^1, R^2$     Alkylen

Vorteil dieses Verfahrens ist, daß sich das Amin und die Alkohol-Komponente in den Produktionskreislauf "Amin → Isocyanat → Polyurethan" zurückführen lassen und somit wieder das gleiche Isocyanat bzw. ein einheitliches Polyurethan hergestellt werden können:

$$n\ R\text{-}NH_2 \Rightarrow n\ R\text{-}NCO + n\ HO\text{-}R^1\text{-}(OR^2)_m\text{-}OH \Rightarrow -[-R\text{-}NH\text{-}(C{=}O)\text{-}O\text{-}R^1\text{-}(OR^2)_m\text{-}O\text{-}]_n\text{-}$$

Alternativ lassen sich vor allem bei hochpreisigen Aminen und Alkoholen diese auch aus dem Kreislauf ausschleusen und für andere chemische Reaktionen verwenden.

Die Hydrolyse von Polyurethanen ist aus der Literatur bekannt (US 4,035,314, US 4,316,992, US 3,441,616). Die dort beschriebenen Verfahren weisen aber eine Reihe von technischen und wirtschaftlichen Nachteilen auf:

Die Hydrolysegeschwindigkeit ist in der Regel gering, so daß lange Reaktionszeiten für einen wirtschaftlichen Umsatz benötigt werden. Bei allgemein notwendigen Reaktionstemperaturen von > 180 °C treten als Folge, verstärkt noch bei Anwesenheit von Katalysatoren, unerwünschte Nebenreaktionen auf, bei denen u. a. unter den Hydrolysebedingungen nichtspaltbare Produkte entstehen.

Weitere Nachteile der beschriebenen Verfahren sind, das zur Hydrolyse überhitzter Wasserdampf bzw. eine Apparatur zu dessen Herstellung zur Verfügung stehen muß. Bei längeren Reaktionszeiten wird das Reaktionsgemisch durch kondensiertes Wasser verdünnt, so daß sich die Notwendigkeit zu dessen kontinuierlichen, teilweise aufwendigen Abtrennung aus dem Reaktionsansatz ergibt. Lösungsmittel und Reaktanden, z. B. Glykol, werden mit dem Wasserdampf ausgetragen und müssen kontinuierlich ergänzt werden.

Die Isolierung der Spaltungsprodukte erfordert aufgrund der Nebenproduktebildung erheblichen technischen, apparativen Aufwand.

Aufgabe der Erfindung war es, ein Verfahren für die Spaltung von Polyurethanen zu finden, das sich durch eine sowohl technisch einfach durchzuführende als auch Nebenprodukt-arme Verfahrensweise auszeichnet und das die oben beschriebenen Nachteile nicht aufweist.

Gegenstand der Erfindung ist ein Verfahren zur Spaltung von Polyurethanen, wobei die zerkleinerten Polyurethane in einem Gemisch aus 10 - 90 Gew.-% Wasser und 90 - 10 Gew.-% Lösemittel, ausgewählt aus ein- oder mehrwertigen aliphatischen oder cycloaliphatischen Alkoholen, Alkylglykolen, Dialkylglykolen, Dialkylglykoldialkylethern, allein oder in Mischungen, bei Temperaturen von mindestens 180°C und einem Druck von mindestens 4 bar gespalten werden, und das der Wasseranteil pro mol zu spaltender Urethan-Bindung des Polyurethans mindestens 1 mol beträgt.

Dazu wird das zerkleinerte Polyurethan in einem Gemisch aus Lösungsmittel und Wasser auf über 180°C erhitzt. Als Lösemittel können ein- oder mehrwertige aliphatische oder cycloaliphatische Alkohole, Alkylglykole, Dialkylglykole, Dialkylglykoldialkylether, allein oder in Mischungen eingesetzt werden. Für das Verfahren geeignete Lösemittel sind z. B. Methanol, Ethanol, Propanole, Butanole, Pentanole, Hexanole, Ethylenglykol, Diethylenglykol, Propylenglykol, Diethylenglykoldialkylether. Als Lösungsmittel wird bevorzugt n-Butanol eingesetzt.

Erfindungsgemäß wird ein Gemisch aus 10 - 90 Gew.-% Wasser und 90 - 10 Gew.-% Lösemittel eingesetzt.

Die Reaktion erfolgt bei mindestens 180 °C, bevorzugt zwischen 200 und 230 °C und einem Druck von mindestens 4 bar, bevorzugt zwischen 4 und 30 bar, besonders bevorzugt zwischen 4 und 10 bar.

Wird als Lösemittel Alkohol oder Glykole verwendet, nimmt mit zunehmender Hydrolyse der Reaktionsdruck ab.

Ein Katalysator, bevorzugt wird Natriumhydroxid eingesetzt, beschleunigt die Hydrolyse. Alkalioxid, Alkalihydroxid, Erdalkalioxid, Erdalkalihydroxid allein oder in Mischungen sind prinzipiell als Katalysatoren geeignet.

Es wurde gefunden, daß das Reaktionsgleichgewicht auf die Produktseite des Amins verlagert und damit die Reaktionszeit verkürzt werden kann, in dem das bei der Reaktion entstehende Kohlendioxid mit einem Inertgas, wie z. B Stickstoff, Helium, Argon, (teil)halogenierte Kohlenwasserstoffe, ausgestrippt wird. Die Nebenproduktebildung ist bei dieser Fahrweise gering. Bevorzugt wird Stickstoff eingesetzt. Das eingesetzte Inertgas kann zurückgewonnen werden.

Nach beendeter Reaktion werden die Spaltkomponenten destillativ aufgearbeitet und getrennt. Bevorzugt werden in einer Vakuumdestillation das Wasser, Lösungsmittel und das gebildete Amin direkt aus dem Reaktionsansatz fraktioniert. Wurde zur Polyurethan-Herstellung eine niedrig siedende Polyol-Komponente eingesetzt, so kann diese ebenfalls destillativ abgetrennt und in die erneute Polyurethanisierung rückgeführt werden. Wasser und Lösungsmittel können in der Regel ohne weitere Aufarbeitungsschritte wieder für die Spaltungsreaktion verwendet werden. Das Amin wird abhängig von der Anforderung einer Feinreinigung unterzogen.

Beispiel:

Das zur Hydrolyse eingesetzte Test-Polyurethan wurde aus folgenden Komponenten hergestellt Als Isocyanat-Komponente wurde Isophorondiisocyanat (IPDI), als Alkohol-Komponenten Trimethylolpropan sowie ein linearer und ein verzweigter Polypropylenglykolether (Voranol®, DOW Chem. Comp.) eingesetzt:

| Komponenten | Gew.-% |
| --- | --- |
| Voranol CP 450 | 30,88 |
| Voranol P 1010 | 30,88 |
| IPDI/ Trimethylolpropan-Addukt<br>IPDI<br>Trimethylolpropan | <br>35,73<br>2,36 |
| Sonstige | 0,15 |
| Summe | 100,00 |

Das zu dünnen Platten ausgehärtete Polyurethan wurde, nach Abkühlung mit Trockeneis, in einer Mühle zu einer Korngröße von ungefähr 3 x 3 x 3 mm gemahlen.

In einem 2 Liter Rührautoklaven wurden 175 g des zerkleinerten Polyurethans mit 300 g n-Butanol und 50 g Wasser auf 230 °C erhitzt. Der Innendruck stieg bis auf max. 20,3 $bar_{abs}$. Durch ein Einleitungsrohr wurde zur Ausstrippung des Kohlendioxids kontinuierlich ein intensiver Stickstoffstrom durch den Reaktor geleitet. Nach 8 h Reaktionsdauer hatte sich das Polyurethan vollständig gelöst und es hatte sich quantitativ Isophorondiamin gebildet. Die Base-Zahl des Reaktionsgemisches lag bei 54 mg KOH/g.

Aus dem Reaktionsansatz wurde anschließend durch Vakuumdestillation zunächst das n-Butanol und Wasser, dann nach Temperaturerhöhung das Isophorondiamin quantitativ abgetrennt. Verunreinigungen im Amin durch Polypropylenglykolether-Anteile müssen in einer Kolonnendestillation im Vakuum entfernt werden.

**Patentansprüche**

1. Verfahren zur Spaltung von Polyurethanen,
**dadurch gekennzeichnet,**
**dass** die zerkleinerten Polyurethane in einem Gemisch aus 10 - 90 Gew.-% Wasser und 90 - 10 Gew.-% Lösemittel, ausgewählt aus ein- oder mehrwertigen aliphatischen oder cycloaliphatischen Alkoholen, Alkylglykolen, Dialkylglykolen, Dialkylglykoldialkylethern, allein oder in Mischungen, bei Temperaturen von mindenstens 180 °C und einem Druck von mindestens 4 bar gespalten werden, und das der Wasseranteil pro mol zu spaltender Urethan-Bindung des Polyurethans mindestens 1 mol beträgt.

2. Verfahren nach Anspruch 1,
**dadurch gekennzeichnet,**
**dass** als Alkohole Methanol, Ethanol, Propanole, Butanole, Pentanole, Hexanole, Ethylenglykol, Diethylenglykol, Propylenglykol, Diethylenglykoldialkylether allein oder in Mischungen eingesetzt werden.

3. Verfahren nach mindestens einem der Ansprüche 1 bis 2,
**dadurch gekennzeichnet,**
**dass** die Spaltung bei Temperaturen von 200 bis 230 °C durchgeführt wird.

4. Verfahren nach mindestens einem der Ansprüche 1 bis 3,
**dadurch gekennzeichnet,**
**dass** die Spaltung bei einem Druck von 4 bis 30 bar durchgeführt wird.

5. Verfahren nach mindestens einem der Ansprüche 1 bis 4,
**dadurch gekennzeichnet,**
**dass** während der Spaltung ein inertes Gas durch die Reaktionsapparatur geleitet wird.

6. Verfahren nach Anspruch 5,
**dadurch gekennzeichnet,**
**dass** als inertes Gas Stickstoff, Helium, Argon, (teil)halogenierte Kohlenwasserstoffe allein oder in Mischungen eingesetzt wird.

7. Verfahren nach mindestens einem der Ansprüche 1 bis 6,
**dadurch gekennzeichnet,**
**dass** ein Katalysator eingesetzt wird.

8. Verfahren nach Anspruch 7,
**dadurch gekennzeichnet,**
**dass** als Katalysator Alkalioxid, Alkalihydroxid, Erdalkalioxid, Erdalkalihydroxid allein oder in Mischungen eingesetzt wird.

9. Verfahren nach mindestens einem der Ansprüche 1 bis 8,
**dadurch gekennzeichnet,**
**dass** die Spaltkomponenten destillativ abgetrennt werden.

10. Verfahren nach mindestens einem der Ansprüche 1 bis 9,
**dadurch gekennzeichnet,**
**dass** das Inertgas rückgewonnen wird.

**Claims**

1. A process for the cleavage of polyurethanes, **characterized in that** the comminuted polyurethanes are cleaved in a mixture of 10-90% by weight of water and 90-10% by weight of solvent, selected from among monohydric or polyhydric aliphatic or cycloaliphatic alcohols, alkyl glycols, dialkyl glycols, dialkyl glycol dialkyl ethers, either alone or in mixtures, at temperatures of at least 180°C and a pressure of at least 4 bar, and the amount of water per mol of urethane bond to be cleaved in the polyurethane is at least 1 mol.

2. A process according to claim 1, **characterized in that** methanol, ethanol, propanols, butanols, pentanols, hexanols, ethylene glycol, diethylene glycol, propylene glycol, diethylene glycol dialkyl ethers, either alone or in mixtures, are used as alcohols.

3. A process according to at least one of claims 1 or 2, **characterized in that** the cleavage is carried out at temperatures of from 200 to 230°C.

4. A process according to at least one of claims 1 to 3, **characterized in that** the cleavage is carried out at a pressure of from 4 to 30 bar.

5. A process according to at least one of claims 1 to 4, **characterized in that** an inert gas is passed through the reaction apparatus during the cleavage.

6. A process according to claim 5, **characterized in that** nitrogen, helium, argon, (partially) halogenated hydrocarbons, either alone or in mixtures, are used as inert gas.

7. A process according to at least one of claims 1 to 6, **characterized in that** a catalyst is used.

8. A process according to claim 7, **characterized in that** alkali metal oxide, alkali metal hydroxide, alkaline earth metal oxide, alkaline earth metal hydroxide, either alone or in mixtures, are used as catalyst.

9. A process according to at least one of claims 1 to 8, **characterized in that** the cleavage components are separated off by distillation.

10. A process according to at least one of claims 1 to 9, **characterized in that** the inert gas is recovered.


**Revendications**

1. Procédé de craquage de polyuréthanes,
   **caractérisé en ce que**
   les polyuréthanes broyés sont craqués dans un mélange constitué de 10 - 90% en poids d'eau et de 90 - 10 % en poids de solvant, choisi parmi des alcools mono- ou polyvalents aliphatiques ou cycloaliphatiques, des alkylglycols, dialkylglycols, dialkyléthers de dialkylglycol, seuls ou en mélanges, à des températures d'au moins 180°C et à une pression d'au moins 4 bars,
   et la proportion d'eau est d'au moins 1 mole par mole de liaison uréthane à décomposer du polyuréthane.

2. Procédé selon la revendication 2,
   **caractérisé en ce que**
   comme alcools, on utilise du méthanol, de l'éthanol, des propanols, des butanols, des pentanols, des hexanols, de l'éthylène glycol, du diéthylène glycol, du propylène glycol, du dialkyléther de diéthylène glycol, seuls ou en mélanges.

3. Procédé selon au moins l'une quelconque des revendications 1 à 2,
   **caractérisé en ce que**
   le craquage est réalisé à des températures de 200 à 230°C.

4. Procédé selon au moins l'une quelconque des revendications 1 à 3,
   **caractérisé en ce que**
   le craquage est réalisé à une pression de 4 à 30 bars.

5. Procédé selon au moins l'une quelconque des revendications 1 à 4
   **caractérisé en ce que**

pendant le craquage, un gaz inerte traverse l'appareil de réaction.

6.  Procédé selon la revendication 5,
    **caractérisé en ce que**
    comme gaz inerte, on utilise de l'azote, de l'hélium, de l'argon, des hydrocarbures (partiellement) halogénés, seuls ou en mélanges.

7.  Procédé selon au moins l'une quelconque des revendications 1 à 6,
    **caractérisé en ce qu'**
    on utilise un catalyseur.

8.  Procédé selon la revendication 7,
    **caractérisé en ce que**
    comme catalyseur, on utilise un oxyde alcalin, un hydroxyde alcalin, un oxyde alcaline-terreux, un hydroxyde alcalino-terreux, seuls ou en mélanges.

9.  Procédé selon au moins l'une quelconque des revendications 1 à 8,
    **caractérisé en ce que**
    les composants du craquage sont séparés par distillation.

10. Procédé selon au moins l'une quelconque des revendications 1 à 9,
    **caractérisé en ce que**
    le gaz inerte est récupéré.